Europäisches Patentamt

**European Patent Office**

**Office européen des brevets**

(11) Publication number: **0 142 172**
**A2**

# EUROPEAN PATENT APPLICATION

(21) Application number: **84113781.3**

(22) Date of filing: **14.11.84**

(51) Int. Cl.⁴: **A 61 K 6/08**
**C 09 J 3/00, C 08 F 299/06**

(30) Priority: **16.11.83 US 552298**

(43) Date of publication of application:
**22.05.85 Bulletin 85/21**

(84) Designated Contracting States:
**AT BE CH DE FR GB IT LI NL SE**

(71) Applicant: **DENTSPLY INTERNATIONAL, INC.**
**570 West College Avenue**
**York Pennsylvania 17405(US)**

(72) Inventor: **Tateosian, Louis H.**
**209 Reynolds Mill Road**
**York Pennsylvania 17403(US)**

(72) Inventor: **Horne, Shek Chee**
**P.O. Box 523**
**Somers Conn. 06071(US)**

(74) Representative: **Wächtershäuser, Günter, Dr.**
**Tal 29**
**D-8000 München 2(DE)**

(54) Composition usable as adhesive.

(57) A new polymerizable composition of matter, especially an adhesive composition that is advantageous as a dental adhesive for bonding with acrylic and metal materials. The composition includes polar organic composition chosen from the group consisting of acids and salts and mixtures thereof, multifunctional vinyl crosslinking composition, and a solvent composition giving said new polymerizable composition solvent action.

The preferred polar organic is an acrylic acid, the preferred multifunctional vinyl crosslinking composition is difunctional acrylic crosslinking monomer, and the solvent preferably includes dicyclopentenyloxyethyl methacrylate. The composition preferably further includes urethane diacrylate oligomer and a polymerization initiator system, preferably visible light curing with an accelerator.

Methods of forming dentures are also disclosed.

Croydon Printing Company Ltd.

0142172

Case
1523
A1/1523
11/16/83

# COMPOSITION USABLE AS ADHESIVE

## Background of the Invention

The present invention relates to a new composition of matter and especially to an adhesive material that bonds to acrylics and metals and that is adaptable for visible light curing in preparing and repairing dentures and dental veneers for the oral environment, especially for humans.

It is well known that adhesives used in denture materials, where they may be exposed to the rigors of the oral environment of the mouth, present many problems. Unless an adhesive is totally encapsulated, its use in the mouth exposes it to the constantly changing rigors of stress from the solvent action of foods to alkaline and acid materials and to severe staining materials. While various acrylic materials, in general, are compatible in adhering to one another, the bonds are not always of an integrity to withstand the long-term thermocycling, tensioning, and twisting involved with mastication of food. Also, the present widely used commercial acrylics require time, temperature, and pressure to effect a bond. The bonding problem is more severe when one of the acrylic materials, i.e. in the instance of the present invention in its preferred embodiment, an acrylic tooth is formed and hardened to a crosslinked structure prior to being engaged by a deformable, moldable acrylic material hardened under ambient conditions, that does not contain a sufficient solvating characteristic for penetrating into the surface of the previously hardened tooth material.

It is an object of the present invention to provide a hardenable adhesive material for durable bonding to an acrylic member.

It is another object of the present invention to provide an environmentally and user safe adhesive material that can be rapidly hardened on command, especially by actinic radiation, under ambient conditions of temperature and pressure.

It is a further object of the present invention to provide an adhesive material for use in bonding acrylic materials and dentures which will not stain or dissolve during prolonged use in the oral environment.

It is a further object of the present invention to provide a hardenable adhesive material for bonding to a metal member.

It is another object of the present invention to provide bonding agents or tie coats useful in the construction and repair of dental appliances, oral prosthetics, and similar articles.

Yet another object of the present invention is to provide a bonding agent functional to bond precured acrylic polymer moldings together.

A further object of the present invention is to provide a bonding agent functional to bond precured acrylic polymer and uncured acrylic compositions that are not ordinarily capable of bonding directly to one another through a single bonding agent.

0142172

## Summary of the Invention

By an aspect of this invention in a preferred form thereof, a new polymerizable composition of matter is provided that includes polar organic composition chosen from the group consisting of acids and salts and mixtures thereof, multifunctional vinyl crosslinking composition, and a solvent composition giving said new polymerizable composition solvent action.

In the preferred new polymerizable composition, the polar organic composition is an acid, preferably acrylic acid; the multifunctional vinyl crosslinking composition is difunctional acrylic crosslinking monomer; and the solvent composition includes dicyclopentenyloxyethyl methacrylate. In a preferred aspect, the composition further includes urethane diacrylate oligomer and a polymerization initiator system including a visible light activated initiator and an accelerator.

The material of the present invention is, in a preferred aspect, an adhesive. In a preferred aspect of the present invention, the adhesive is operable as a bonding composition in a method of forming a denture that includes forming a tooth structure of ridged acrylic, coating at least a portion of the tooth structure with the bonding composition and engaging an acrylic denture base composition with said coating.

In another aspect of the present invention, the bonding composition bonds an acrylic veneer to a metal bridge substructure and is cured through the acrylic veneer using radiant energy, preferably visible light.

## Description of the Preferred Embodiment

The present invention in its preferred embodiment is a new composition of matter suitable for use with an initiator system that enables a quick curing, bonding of the composition. This new composition of matter in one preferred embodiment functions as an adhesive material for bonding two acrylic materials together. The new adhesive composition can be applied first to the surface of an already hardened, crosslinked acrylic member where it can penetrate the hardened surface and thereafter be cured to form a tie coat firmly interlocked with the hardened surface. This tie coat in a preferred embodiment has a substantial reactive air inhibited surface for subsequent attachment thereto of an unhardened acrylic material. In the preferred form of the present invention, the second acrylic material is in the form of a putty-like moldable substance that can be conformed closely against the tie coat and has sufficiently fluidity to penetrate into and swell the structure of the tie coat with facility, but not enough fluidity to adequately bond to the original hardened acrylic member.

The new composition of matter is in its essential elements: a polar organic composition, a multifunctional vinyl crosslinking composition, and a solvent composition. Preferably, a urethane diacrylate oligomer is included. The preferred solvent composition includes dicyclopentenyl-oxyethyl methacrylate. The composition also preferably contains a photopolymerizing actinic light initiator system that upon activation causes the functional compounds to polymerize into a crosslinked structure.

The preferred polar organic compositions are those chosen from the group consisting of acids, salts, and mixtures thereof. Preferably, the polar organic compositions are copolymerizable. The most preferred polar organic compositions are the acids.

The preferred acid is an acid that is polymerized with other components of the new composition of matter. The more preferred acids are the acrylic acids, preferably present in the new composition in an amount of 0.1 to 30 weight percent, more preferably 1 to 10, and most preferably 3 to 9 weight percent. Preferably the acrylic acid is acrylic or methacrylic acid and most preferably acrylic acid.

The multifunctional vinyl crosslinking monomer composition is preferably a difunctional acrylic composition. The difunctional acrylic crosslinking monomeric composition is preferably present in the new composition in an amount of 0.1 to 30 weight percent, more preferably 0.5 to 20, and most preferably 2 to 15 weight percent. The difunctional acrylic crosslinking monomeric composition is preferably a compound that is a monomer including the oligomers of low molecular weight which have repeating units totaling from $C_2$ to $C_{10}$ (two carbons to ten carbons). Especially preferred are the dimethacrylate esters, more preferably the di, tri, and tetraethylene dimethacrylates which are ethylene glycol methacrylate esters and its oligomers and aliphatic diesters.

It is an important feature of the present invention that the new composition of matter include a composition that gives the new composition of matter a solvent action when the composite composition is applied to another material, i.e. structure or substrate. When the new composition of matter, composite composition, is applied for bonding to crosslinked acrylics as is the preferred purpose of the present invention in one of its aspects, complete solution cannot take place, but instead solution of polymer chain segments is desired and this results in swelling. The swelling allows interpenetration of the composite composition into the crosslinked structure, which upon curing, effects a good bond. It is especially advantageous for the bonding agent to contain compounds that have solubility parameters of 8.5 to 13.3 $(cal/cm^3)^{\frac{1}{2}}$ as described in Polymer Handbook Editors Branchup and Immergut, Interscience Publishers 1966, Chapter IV, page 344 to 368. Many of the solvent compounds useful for this invention are included in Table 2 of Chapter IV of the above reference, especially those that are weakly or moderately hydrogen bonded and excluding those that are strongly hydrogen bonded. Preferred compounds include methyl methacrylate with solubility parameter 8.8, methylene chloride with solubility parameter 9.7, and dicyclopentenyloxyethyl methacrylate with solubility parameter 8.6 or mixtures thereof.

It is an especially preferred feature of the present invention that the solvent composition include the compound dicyclopentenyloxyethyl methacrylate and preferably in an amount of at least 20 weight percent of the total solvent composition. The other solvent is preferably present with the dicyclopentenyloxyethyl methacrylate in an amount of at least 25 weight percent of the total solvent content. More preferably, the solvent is at least 25 weight percent dicyclopentenyloxyethyl methacrylate with other organic solvent composition being at least 30 weight percent of other organic solvent compositions. The total solvent composition is preferably present in an amount of 5 to 80 weight percent, more preferably 20 to 60 weight percent, and most preferably 30 to 50 weight percent of the composite composition. The dicyclopentenyloxyethyl methacrylate provides an unexpectedly effective solvent creating effect on the composite composition. Also surprising is the rapid polymerization effected in the composite composition containing dicyclopentenyloxyethyl methacrylate.

The preferred new composition of matter of the present invention includes a urethane diacrylate oligomer, and is preferably present in the new composition in an amount of 20 to 70 weight percent, more preferably 35 to 60, and most preferably 40 to 50 weight percent. The preferred urethane diacrylates are formed from polyester diols and polyether diols that are end capped with aromatic, aliphatic, or cycloaliphatic diisocyanates and then acrylated. Alternatively, the preferred urethane diacrylates are formed from aliphatic or aromatic diisocyanates coupled with

0142172

hydroxyethyl methacrylate or hydroxypropyl methacrylate.

Especially preferred are the urethane diacrylates formed from polyalkyl glycol capped with adipic acid; then capped with an aromatic diisocyanate to form a second diisocyanate with average molecular weight of 4,000 to 5,000. This second diisocyanate is then acrylated to form the urethane diacrylate especially preferred.

The preferred polymerization initiator system composition is a radiation activatable composition. In some instances, a heat activatable initiator compound may also be included. More preferably, the radiation activatable composition is an actinic light activatable composition or photoinitiator system. Such compositions are well-known; see, for example, United States Patent Number 4,245,031, the contents of which are incorporated herein by reference.

Most preferably, the initiator system includes a visible light activatable compound making the polymerizable composition curable with visible light. The visable light activated photoinitiator is preferably present in an amount of 0.01 to 10 weight percent, more preferably 0.05 to 5, and most preferably 0.1 to 1 weight percent. The preferred class of initiators is the well-known -diketones and the most preferred is camphoroquinone. Visible light activatable photoinitiators are discussed in United States Patent Number 4,110,184, the contents of which are incorporated herein by reference.

The preferred initiator system includes an accelerator. The preferred accelerator is an organic amine. The

0142172

most preferred accelerators are the organic amine salts that are the subject of United States Patent Application, Serial Number 486,688, the contents of which are incorporated herein by reference. The most preferred organic amine salts are those containing a dimethyl tertiary amine moeity with a neopentyl acrylate radical and in which the amine salt is of an organic acid and the preferred acid is methacrylic acid.

The photoinitiator system composition, including both the initiator composition and the accelerator composition, is preferably present in the composite composition in an amount of 0.01 to 50 weight percent, more preferably 0.2 to 25 weight percent, and most preferably 0.4 to 5 weight percent.

It is often desirable to add additional materials to the composite composition of the present invention, such materials as stabilizers , thickeners, pigment fillers, wetting agents, and adhesion promoters. In addition, inherent impurities, stabilizers, processing aids, and the like are frequently present in the various component ingredients.

The present invention in its preferred form is the adhesive material and has a special utility for bonding to an acrylic member such as the dental appliances disclosed in United States Patent 4,396,377 and assigned to the assignee of the present application.

The preferred denture base materials are visible light curable, and preferred in the sense of the need for the present invention are those that are filled containing fillers such as inorganic and organic fillers. Example fillers are silaneous fillers and other fillers by way of example such as apatite, soda glass, quartz, silica gel, borosilicate glass, aluminum, metal fibers, polymeric fibers, and particularly polymer particles. These fillers may be in the form of spheres, platelets, fibers, whiskers, or may be irregularly shaped. The fillers may be present in quantities of 35 to 70 percent by volume of the denture base material. As a general consideration, fillers are selected to minimize interferes with the passage of radiation.

The polymerizable organic compounds of the preferred denture base materials are preferably ethylenically unsaturated and preferably, additionally polymerizable and most preferably acrylate monomers, especially as shown in United States Patent Application Serial Number 486,688, filed April 26, 1983, which application is incorporated herein by reference. Preferably, the organic compound is present in an amount of about 1 to about 99.9 percent by weight of the polymerizable system. The polymerizable system is made up of one or more of the following materials: polymerizable organic compound or compounds, the initiator material, and the accelerator material.

The present invention has especial utility for bonding acrylic teeth to visible light curable denture base materials and repairing broken dentures including those that have missing pieces. In the instance where pieces are missing, the bonding agent may be used as an adhesive primer or tie coat to bond a visible light curable repair portion

**0142172**

of denture base material. Additionally, the invention has utility in providing tie coats between metal dental bridgework and veneer materials for new bridgework and for repair of porcelain veneered metal bridgework in the mouth. The solvent action of the composite composition enables the thorough penetration of the films and deposits on the surfaces of the metal, particularly oily films. An excellent bond can be achieved.

The invention may be used in a method of forming a plastic veneered dental bridge by forming a bridge substructure, coating the metal with the bonding composition and then engaging the bonding composition with an acrylic veneer material. The bonding composition is then preferably cured by means of an included radiation activatable compound being activated. Preferably, the curing system is the visible light curing system described and the light is projected through the acrylic veneer. A light opaquing agent is preferably included in the bonding composition to mask the metal for an aesthetic dental construction.

All percentages in this patent application are weight percents based on 100 percent of the final composition except where clearly indicated as percents of additive combinations or of recited compositions. Where indicated as a percent of recited compositions or components, it is meant that the materials recited in the series are to be apportioned among themselves to equal 100 percent when added up. This means, for example, a large amount of filler could be present and not used in the calculation. Unless another meaning is clearly intended, compound is meant to define a specific chemical compound while composition is meant to define one or more compounds having a stated characteristic either singly or in combination. A composite composition is a composition including two or more compositions differing in stated characteristic. A polymerizable composition is one that can polymerize. As a general understanding, the polymerizable compositions of the present invention harden on polymerization, turning in most preferred instances from liquids to solids that have firm attributes of hardness or shape retaining character, generally having some rigidity with flexing ability.

The invention is further illustrated by the following examples:

### Example 1

A visible light curable bonding agent composition or adhesive tie coat of the present invention was prepared by charging to a glass reactor the ingredients listed below in the proportions given and in the manner described below in a red light room.

0142172

| Percent | Ingredient |
|---------|-----------|
| 21.58% | Dicyclopentenyloxyethyl methacrylate (QM 657) Rohm and Haas Company |
| 4.32% | Acrylic Acid |
| 7.19% | 1,6-hexanediol dimethacrylate (HDDMA) |
| 0.31% | Camphoroquinone (CQ) |
| 1.61% | Dimethylaminoneopentyl acrylate - methacrylic acid (DMANPA-MAA) salt* |
| 19.66% | Methymethacrylate (MMA) |
| 45.33% | Acrylated urethane oligomer (Uvithane 782, Morton-Thiokol Corporation) |

*DMANPA-MAA salt comprising 53.21g DMANPA, 26.73g MAA, 0.46g BHT prepared as described in following organic amine salt preparation procedure.

The CQ was dissolved in the HDDMA and then added to the reactor. Next, the acrylic acid, DMANPA-MAA, QM657, and MAA were added with stirring. Finally, the Uvithane 782, previously heated to 60°C for three hours, was added to the mixture with stirring. The mixture was stirred for 2.5 hours. A visually uniform solution was obtained. The solution was transferred to light proof bottles.

### Organic Amine Salt Preparation

An organic amine salt was prepared from two feedstocks as follows:

332.1g glacial methacrylic acid (MAA) and 5.7g BHT were added to a two liter polyethylene bottle. The mixture was agitated until the BHT dissolved into a uniform solution. The MAA solution was then placed in a -12°C freezer for four hours. The solution froze to a solid. At substantially the same time, about 700g of dimethylaminoneopentyl acrylate (DMANPA) was added to a one liter polyethylene

0142172

bottle and placed in the freezer for four hours. The DMANPA did not freeze and remained liquid.

Both bottles were removed from the freezer and 662.2g of DMANPA were poured onto the frozen MAA solution. The combined MAA and DMANPA were mixed on a roller mill for one hour. The resulting amine salt DMANPA-MAA was a clear, slightly viscous liquid.

### Bonding Procedure

A set of arch form teeth was formed according to the teaching of United States Serial Number 510,405, filed July 1, 1983 and assigned to the same assignee as the present application, the contents of which application are incorporated herein by reference, using the teaching of United States Patent .4,396,377 previously referred to in this application.

A moldable denture base composition was formed that is hardenable with visible light. The denture base material was prepared according to the following formula:

### Percent by Weight of Total Composition

| | |
|---|---|
| 39.01 | Urethane dimethacrylate (reaction product of hydroxyethyl methacrylate and 2,2,4-trimethyl-hexyl-1, 6-diisocyanate) |
| 2.54 | 1,6-hexanediol dimethacrylate (HDDMA) |
| 0.13 | Camphoroquinone (CQ) |
| 0.66 | DMANPA-MAA salt |
| 42.48 | Poly(methyl methacrylate-co-ethylene dimethacrylate 99.8:0.2) a polymer supplied by L. D. Caulk Company* |
| 0.08 | Red acetate fibers |
| 0.02 | Pigments |
| 14.92 | Fumed silica inorganic filler (Aerosil R972, a product of DeGussa) |
| 0.16 | Gamma-methacryloxypropyltrimethoxysilane |

*The polymer was prepared according to the teaching of United States Patent 4,396,476.

0142172

First, the CQ was dissolved in HDDMA and then mixed with the amine salt, gamma-methacryloxypropyltrimethoxysilane, and urethane dimethacrylate. The resulting liquid solution was charged to a double planetary mixer heated to 45°C and mixed under 20 mm Hg pressure. Next the polymer with the pigments and fibers previously blended in a V-Cone Blender was added and mixed under 20 mm pressure. The temperature was increased to 55°C and the fumed silica was added in three increments of about equal size and mixed under 130 mm pressure each time. This produced a visible light curable (VLC) putty-paste.

The VLC putty was molded into a sheet 3.5 inches x 2.5 inches x 0.10 inches in a hydraulic press. The sheet was adapted as a baseplate to a stone model (coated with separator) made from an impression of the mouth. The baseplate was trimmed and then cured in two minutes on a turntable rotating under four 150 watt quartz-halogen lamps with a 400 to 500 nm band-pass filter. A preferred apparatus is shown in United States Serial No. 492,284, filed May 6, 1983. The light flux varied from 100 to 130 mw/cm² on the surface of the baseplate. Additional VLC putty was rolled into rope 0.25 inches in diameter. The rope was adapted around the ridge of the baseplate in a configuration to receive the pressed-in full arch of acrylic plastic teeth.

## Application of Bonding Agent

The bonding agent composition formulated, as described earlier in this example, was applied as a visually thorough and complete covering to the ridge lap areas and about 2 mm onto the facial, lingual collar and interproximal areas of full arch upper teeth by applying with a brush. The bonding agent was bench set for two minutes and then light cured two minutes in the cure unit previously described.

The teeth coated with the bonding agent were then press-positioned in the rope of VLC putty. The teeth were then further trued in position in an articulator and then fixed in position by a two-minute light cure. Next the facial and lingual aspects of the denture were finished with additional rope.

A liquid oxygen barrier coating described below was applied as a top coating before curing the visible light curable denture resin. The surface after photocuring for four minutes was dry, shiny, and tack free. The denture was then removed from the stone model and other side of the denture was coated with the liquid oxygen barrier coating and then cured for two minutes under the light. The surface was dry, shiny, and tack free. Next, the cured denture was washed with tap water and dried with a paper towel. Both surfaces of the processed denture were shiny and tack free.

The oxygen barrier layer was prepared according to the following formulation:

| | |
|---|---|
| 41.00% | Polyvinyl pyrrolidone (Plasdone K 29/32, GAF Corporation) |
| 58.53 | Water |
| 0.045 | Silicone antifoam (SAG 471, Union Carbide Corporation) |
| 0.225 | Surfactant (Makon 10, Stepan Chemical Corporation) |
| 0.20 | Potassium sorbate |

The above ingredients were mixed together at ambient conditions and the resulting solution was a very viscous clear yellow colored liquid with a viscosity of 850 centipoises at 22°C (Brookfield #2 spindle, 20 rpm).

### Example 2

This example demonstrates the usefulness of the VLC bonding agent to repair the previously described VLC denture base material by bonding pieces together.

The previously described VLC denture base material was pressed into a 120 mm x 90 mm x 7 mm deep brass mold with three cavities, each measuring 70 mm x 13 mm x 7 mm deep. The brass mold was positioned on top of an aluminum back-up strip with a cellophane film between the brass and the aluminum. Another cellophane film was pressed on top of the VLC denture base material to form three bars. The VLC denture base material was then cured for five minutes on each side using four 150 watt quartz-halogen lamps at a distance of 15 cm from the lamps (50 mw/cm² in the 400 to 500 nm region).

After removal from the mold, the cured VLC denture base material bars were cut in half widthwise using a low speed saw, and then washed clean with soap and water, rinsed, and dried.

A bonding agent was prepared according to the procedure using the specific materials described in Example 1 using the following formulation:

|        |                                         |
|--------|-----------------------------------------|
| 15.00g | Dicyclopentenyloxyethyl methacrylate    |
| 31.50g | Uvithane 782                            |
| 3.00g  | Acrylic acid                            |
| 5.00g  | HDDMA                                   |
| 0.15g  | CQ                                      |
| 0.78g  | DMANPA-MAA salt                         |
| 15.00g | MMA                                     |

0142172

The VLC bonding agent was brushed onto one end of the cured VLC denture resin bars, and the other half of the cured VLC denture resin bars were placed onto the bonding agent. The assembly was bench set for two minutes, then cured for five minutes on each side using the curing unit described above. The samples were trimmed on a Buehler wheel using 240 grit sand paper and then immersed in 37°C water for forty-four hours. Five samples were then tested by pulling them in tension using a Universal Instron Testing Machine at a crosshead speed of 0.1 inch/minute. The average bond strength for five tests was 2,575 ± 704 psi.

### Example 3

This example demonstrates the usefulness of the VLC bonding agent of Example 2 for repairing compression packed heat cured denture resin, such as Compak 20 , a product of Dentsply International. The procedure of Example 2 was repeated except for the preparation of the denture resin specimens as described. Samples measuring 70 mm x 13 mm x 7 mm were processed from Compak 20 by using a plaster mold in a brass flask. The compression packed denture resin was mixed as directed, bench set for twenty minutes, and then cured for twenty minutes in boiling water. The flask was then cooled in air for twenty minutes, in water for twenty minutes and then deflasked. The bars were cut in half and the bonding agent of Example 2 was brushed onto the bonding surface of the cured Compak 20 bar; another cured Compak 20 bar was then placed onto the coated surface. The assembly was bench set for two minutes at ambient conditions before curing for five minutes on each side as in Example 2.

The average bond strength of the five samples was 1,438 ± 502 psi.

## Examples 4-20

A series of Examples were conducted in which bonding agent compositions were prepared according to the teaching of Example 1 and tested according to the teaching of Example 2 except with the variations indicated.

TABLE I

| Composition | Example 4 | Example 5 | Example 6 | Example 7 |
|---|---|---|---|---|
| Dicyclopentenyloxyethyl methacrylate | 30.00g | 30.00g | 30.00g | 30.00g |
| Acrylated urethane oligomer | 62.14 | 62.14 | 62.14 | 62.14 |
| Acrylic acid | 0 | 0 | 0 | 0 |
| HDDMA | 0 | 0 | 0 | 0 |
| Methyl Methacrylate | 10.00 | 20.00 | 30.00 | 40.00 |
| Camphoroquinone | 0.30 | 0.30 | 0.38 | 0.30 |
| DMANPA-MAA salt | 1.56 | 1.56 | 2.00 | 1.56 |
| Bond Strength* in psi (Standard Deviation) | 489 (104) | 855 (72) | 804 (417) | 1045 (177) |
| Coffee Stain (2 hours boiling coffee) | light-moderate | light-moderate | light-moderate | light-moderate |

*An acrylic tooth material prepared according to the teaching of United States Patent 4,396,377 was molded into a rectangular bar measuring 1/4 inch x 1/2 inch x 2 3/4 inches and cured five minutes at 250°F. The bars were cut in half and the end of the bar was ground and coated with VLC bonding agent, allowed to bench set two minutes, then cured two minutes under a quartz halogen lamp as described in Example 3 of our previous patent application (United States Serial No. 492,441). A VLC denture base bar measuring 1/4 inch x 1/2 inch x 1 3/8 inch was then applied and light cured four minutes. The rectangular cross section bond specimens thus constructed were conditioned in 37°C water for 188 hours and then tested in tension at room temperature on an Instron Universal Testing Machine using a crosshead speed of 0.1 inch per minute. Five samples of each material were tested.

The examples in Table I show that visible light curable bonding agents consisting of dicyclopentenyloxyethyl methacrylate, acrylated urethane oligomer, methyl methacrylate, camphoroquinone, and DMANPA-MAA salt are effective tooth bonding agents, but were only moderately stain resistant. The bond strength increased with increasing methyl methacrylate content.


TABLE II


| Composition | Example 5 | Example 8 | Example 9 | Example 10 | Example 11 |
|---|---|---|---|---|---|
| Dicyclopen-tenyloxyethyl methacrylate | 30.00g | 30.00g | 30.00g | 30.00g | 0    g |
| Acrylated urethane oligomer | 62.14 | 62.14 | 62.14 | 62.14 | 62.14 |
| Acrylic acid | 0 | 2.00 | 4.00 | 6.00 | 16.00 |
| HDDMA | 0 | 0 | 0 | 0 | 0 |
| Methyl Methacrylate | 20.00 | 20.00 | 20.00 | 20.00 | 20.00 |
| Camphoro-quinone | 0.30 | 0.30 | 0.30 | 0.30 | 0.30 |
| DMANPA-MAA salt | 1.56 | 1.56 | 1.56 | 1.56 | 1.56 |
| | | | | | |
| Bond Strength in PSI (Standard Deviation) | 855* (72) | 1381** (276) | 1227** (382) | 1035** (393) | Not tested due to higher degree of stain |
| | | | | | |
| Coffee Stain (2 hours boiling coffee) | light-moderate | light-moderate | light-moderate | light-moderate | moderate-severe |

*Samples conditioned in 37°C water for 188 hours before testing at room temperature.
**Samples conditioned in 37°C water for 68 hours before testing at room temperature.

The examples of Table II illustrate the effect of acrylic acid content on the bond strength and stain resistance. The bond strength of VLC bonding agents of Examples 8 and 9 containing acrylic acid are significantly higher than the composition of Example 5 that contains no acrylic acid. Example 11 illustrates that the stain resistance of bonding agents decreases at the higher acrylic acid content of 16 percent.

Table III

| Composition | Example 12 | Example 13 | Example 14 | Example 15 |
|---|---|---|---|---|
| Dicyclopenteny- loxyethyl methacrylate | 30.00g | 30.00g | 30.00g | 30.00g |
| Acrylated ure- thane oligomer | 62.14 | 62.14 | 62.14 | 62.14 |
| Acrylic acid | 6.00 | 6.00 | 6.00 | 6.00 |
| HDDMA | 0 | 0 | 0 | 0 |
| Methyl meth- acrylate | 10.00 | 20.00 | 30.00 | 40.00 |
| Camphoroquinone | 0.30 | 0.30 | 0.30 | 0.30 |
| DMANPA-MAA salt | 1.56 | 1.56 | 1.56 | 1.56 |
| Bond Strength in psi (Standard Deviation) | 1044* (260) | 1035** (393) | 1358* (54) | 1237* (148) |
| Coffee Stain (2 hours Boiling Coffee) | light-moderate | light-moderate | light-moderate | light-moderate |

*Samples conditioned in 37°C water for 188 hours before testing.
**Samples conditioned in 37°C water for 68 hours before testing.

The examples of Table III illustrate that VLC bonding agents with the combination of acrylic acid and methyl methacrylate give excellent bond strength while retaining light to moderate stain resistance.

- 23 -

0142172

Table IV

| Composition | Example 14 | Example 16 | Example 17 | Example 18 | Example 19 | Example 20 |
|---|---|---|---|---|---|---|
| Dicyclopen-tenyloxyethyl methacrylate | 30.00g | 30.00g | 30.00g | 30.00g | 30.00g | 30.00g |
| Acrylated urethane oligomer | 63.00 | 63.00 | 63.00 | 63.00 | 63.00 | 63.00 |
| Acrylic acid | 6.00 | 6.00 | 6.00 | 6.00 | 6.00 | 6.00 |
| HDDMA | 0 | 2.00 | 4.00 | 6.00 | 8.00 | 10.00 |
| Methyl methacrylate | 30.00 | 30.00 | 30.00 | 30.00 | 30.00 | 30.00 |
| Camphoro-quinone | 0.30 | 0.30 | 0.30 | 0.30 | 0.30 | 0.30 |
| DMANPA-MAA salt | 1.56 | 1.56 | 1.56 | 1.56 | 1.56 | 1.56 |
| Bond Strength in psi | 1358* | 1378** | 1190** | 1139** | 1319** | 1301** |
| (Standard Deviation) | (54) | (245) | (176) | (362) | (446) | (337) |
| Coffee Stain (2 hours boiling coffee) | light-moderate | light-moderate | light | light | light | very light |

*Samples conditioned in 37°C water for 188 hours before testing.
**Samples conditioned in 37°C water for 68 hours before testing.

The examples of Table IV illustrate that the use of 1,6-hexanediol dimethacrylate increases the stain resistance of the VLC bonding agent without significantly deteriorating their excellent bond strengths. The bond strengths of Examples 18, 19, and 20 containing methyl methacrylate are about twice that of similar compositions of Examples 29, 30, and 31 containing methylene chloride.

### Example 21

A bonding agent composition was formed according to the teaching of Example 1 except with the variations indicated.

The formulation was:

| Percent by Weight of Total Composition | Ingredient |
| --- | --- |
| 70.27g | Urethane dimethacrylate |
| 0.21g | CQ |
| 1.41g | DMANPA-MAA salt |
| 28.11g | Methylene chloride |

The above bonding agent was brushed onto the surface of flat blocks made of plastic tooth materials prepared according to the teaching of United States Patent 4,396,377. The tooth block was set on the bench for two minutes to evaporate the solvent. It was then placed under a 150 watt quartz-halogen lamp and cured for two minutes. A layer of the visible light curable denture resin was then placed onto the coated plastic tooth block, and cured for four minutes under the lamp. A screwdriver was used to push off the denture resin from the tooth block. There was practically no bond between the denture resin and the tooth material.

## Examples 22-25

Bonding agent compositions were formed according to the teaching of Example 1 and were tested according to the teaching of Example 21, except with the variations indicated.

The formulations were:

| Composition | Example 22 | Example 23 | Example 24 | Example 25 |
|---|---|---|---|---|
| Urethane dimethacrylate | 49.00% | 49.09% | 39.27% | 29.45% |
| Dicyclopenteny- loxyethyl methacrylate | 21.00 | 49.09 | 58.90 | 68.72 |
| CQ | 0.21 | 0.29 | 0.29 | 0.29 |
| DMANPA-MAA salt | 1.09 | 1.53 | 1.53 | 1.53 |
| Methylene chloride | 28.63 | 0 | 0 | 0 |
| BHT | 0.07 | 0 | 0 | 0 |
| Bond strength by push- off test | fair- good | fair | fair | fair- poor |
| Ratio urethane dimethacrylate/ dicyclopenteny- loxyethyl meth- acrylate | 70/30 | 50/ | 40/60 | 30/70 |

The bond test was carried out in the same manner described in Example 23. There was fair to good bond strength between the denture base material and the plastic tooth material for Example 24. Comparison of Example 24 with Example 23 illustrates that dicyclopentenyloxyethyl methacrylate enhances the adhesive property of the bonding agent.

The ratio of urethane dimethacrylate/dicyclo- pentenyloxyethyl methacrylate was varied in Examples 24-27 from 70/30 to 50/50, 60/40, and 30/70. The bond strength decreases with increasing levels of dicyclopentenyloxyethyl methacrylate. This indicated that the ratios of 50/50 and higher were preferred. The most preferred was about 70/30.

### Examples 28-30

Bonding agent compositions of Example 36-38 were formed according to the teaching of Example 1 and tested according to the teaching of Example 21 except with the variations indicated.

The formulations were:

| Composition | Example 28 | Example 29 | Example 30 |
|---|---|---|---|
| Urethane dimethacrylate | 46.98 | 47.64 | 48.31 |
| Dicyclopentenyloxyethyl methacrylate | 20.14 | 20.42 | 20.70 |
| CQ | 0.20 | 0.20 | 0.20 |
| DMANPA-MAA salt | 1.05 | 1.06 | 1.08 |
| Methylene chloride | 27.45 | 27.83 | 28.23 |
| Acrylic acid | 4.11 | 2.78 | 1.41 |
| BHT | 0.07 | 0.07 | 0.07 |
| | | | |
| Bond strength by push-off test | excellent | fair-good | fair-good |
| Stain after 4 hours in boiling coffee | severe | severe | severe |

Examples 28-30 illustrate that increased bond strength is obtained by incorporation of acrylic acid, but staining is severe even at the lowest level.

### Examples 31-38

The bonding agent compositions of Examples 31-38 were formed according to the teaching of Example 1 except with the variations indicated.

The formulations were:

## Composition in (g) Grams

| | Ex. 31 | Ex. 32 | Ex. 33 | Ex. 34 | Ex. 35 | Ex. 36 | Ex. 37 | Ex. 38 |
|---|---|---|---|---|---|---|---|---|
| Urethane dimethacrylate | 22.42 | 22.08 | 21.76 | 21.43 | 21.13 | 20.82 | 20.53 | 20.25 |
| Dicyclopentenyloxyethyl methacrylate | 47.07 | 46.38 | 45.71 | 45.01 | 44.36 | 43.72 | 43.11 | 42.52 |
| Acrylic acid | 4.48 | 4.42 | 4.35 | 4.28 | 4.23 | 4.16 | 4.11 | 4.05 |
| 1,6-hexanediol dimethacrylate | 1.49 | 2.94 | 4.35 | 5.71 | 7.04 | 8.33 | 9.58 | 10.80 |
| CQ | 0.22 | 0.22 | 0.22 | 0.23 | 0.23 | 0.24 | 0.23 | 0.24 |
| DMANPA-MAA salt | 1.17 | 1.15 | 1.13 | 1.20 | 1.18 | 1.21 | 1.22 | 1.21 |
| Methylene chloride | 28.08 | 22.74 | 22.41 | 22.07 | 21.76 | 21.45 | 21.15 | 20.86 |
| BHT | 0.07 | 0.07 | 0.07 | 0.07 | 0.07 | 0.07 | 0.07 | 0.07 |
| Bond strength between VLC denture base and unground tooth material by push-off test | excellent | very good | very good | very good | very good | fair | poor | poor |
| Bond strength between VLC denture base and ground tooth material by push-off test | excellent | excellent | excellent | excellent | excellent | good | fair | poor |
| Degree of staining after 4 hours in boiling coffee | moderate-severe | moderate | light | light | very light | very light | very light | extremely light |

*The bonding agents of Examples 33, 34, and 35 were tested for bond strength between VLC denture base and acrylic tooth material using a butt joint tensile bond test. A rectangular specimen of tooth material was lightly ground and the VLC bonding agent was applied to the ground surface and cured. Next, VLC denture base was applied to the cured bonding agent and cured as described in Example 1. The rectangular butt joint samples (0.1376 square inch cross section) were conditioned in 37°C water for six days, then tested in tension on an Instron Universal Testing

Machine using a head speed of 0.1 inch per minute. The bond strengths obtained for the bonding agents described in: Example 30 was 681 psi; Example 31 was 630 psi; and Example 32 was 584 psi.

These bond strengths permit adhesive bonding of acrylic denture teeth to the supporting denture base without dependence on the mechanical retention as used for porcelain teeth. The bonding agents of Examples 3, 31, and 32 disclosed further prohibits the discoloration due to seepage and this avoids producing dentures that are weaker and aesthetically less desirable. The bonding agents disclosed herein eliminate these problems.

The bond strength and stainability of Examples 28-35 decrease with an increase in the 1,6-hexanediol dimethacrylate content. Examples 33-35 give the best balance of bond strength and stain resistance.

## Example 39

A bonding agent composition was formed according to the teaching of Example 1 except with the variations indicated.

The formulation was:

### Composition

| | |
|---|---|
| Urethane dimethacrylate | 56.15 |
| CQ | 0.19 |
| DMANPA-MAA salt | 18.71 |
| Methylene chloride | 24.95 |

| | |
|---|---|
| Bond strength by push-off test | very good |
| Stain after 3 hours in boiling coffee | severe |

This example illustrates that the polar compound DMANPA-MAA salt contributes to the bond strength and stainability much like acrylic acid.

- 29 -                                    0142172

## Examples 40-41

Adhesive opaquers for bonding to crown and bridge metal alloys were mixed in a waring blender at ambient temperature.

### Percent By Weight of 100%

| Composition | Example 40 | Example 41 |
|---|---|---|
| Ethylene dimethacrylate | 10.04 | 9.57 |
| Dicyclopentyl methacrylate | 10.04 | 9.57 |
| 1,6-hexanediol dimethacrylate | 25.99 | 27.71 |
| Ethoxylated bisphenol A dimethacrylate | 10.04 | 9.57 |
| 4-methacryloxyterphthalic acid (4-META) | 0 | 4.72 |
| Methacrylic acid | 8.05 | 4.72 |
| CQ | 0.30 | 0.28 |
| Dimethylaminoethyl methacrylate | 0.89 | 0.85 |
| Elvacite 2028 (A product of Duport) | 29.89 | 27.26 |
| Aerosil 200 (A product of DeGussa) | 0.99 | 0 |
| Titaniumdioxide | 3.21 | 4.89 |
| Iron oxide | 0.03 | 0.05 |
| Aerosil R972 (A product of DeGussa) | 0.53 | 0.81 |
| Lap shear bond strength (psi) between Prisma-fil, a product of Dentsply, and plated Biobond C&B Alloy, a product of Dentsply | 283 | 509 |

The lap shear bond strength of the opaquers was run by the procedure described in Example 6 of United States Serial No. 370,215, filed April 20, 1982.

Examples 40 and 41 illustrate that the 4-META is an effective adhesion promoter for bonding to dental alloys. The use of 4-META, in combination with methacrylic acid in Example 41, gives nearly double the bond strength of methacrylic acid alone in Example 40.

**0142172**

     While in accordance with the patent statutes what is at present considered to be the preferred embodiment of the invention has been described, it will be obvious to those skilled in the art that numerous changes and modifications may be made therein without departing from the invention, and it is therefore aimed in the appended claims to cover all such equivalent variations as fall within the true spirit and scope of the invention.

0142172

It is Claimed:


1.  A new polymerizable composition of matter comprising:

   a.  polar organic composition chosen from the group consisting of acids and salts and mixtures thereof,

   b.  multifunctional vinyl crosslinking composition, and,

   c.  a solvent composition giving said new polymerizable composition solvent action.


2.  The new polymerizable composition of Claim 1 wherein said polar organic composition is an acid and is present in an amount of about 0.1 to about 30 weight percent of the recited components, said multifunctional vinyl crosslinking composition is difunctional acrylic crosslinking monomer composition present in an amount of about 0.1 to about 30 weight percent of the recited components, and said solvent composition is present in an amount of about 5 to about 80 weight percent of the recited components and wherein said new polymerizable composition further comprising urethane diacrylate oligomer composition present in an amount of about 20 to about 70 weight percent of the recited components and polymerization initiator system composition present in an amount of about 0.01 to about 50 weight percent of the recited components.

3. The new polymerizable composition of Claim 1 wherein said solvent comprising dicyclopentenyloxyethyl methacrylate.

4. The new polymerizable composition of Claim 3 wherein said solvent comprising at least about 20 weight percent dicyclopentenyloxyethyl methacrylate and at least about 25 weight percent of other organic solvent composition.

5. The new polymerizable composition of Claim 4 wherein said other organic solvent compound is chosen from the group consisting of methyl methacrylate and methylene chloride.

6. The new polymerizable composition of Claim 2 wherein said initiator system comprises a radiation activatable compound.

7. The new polymerizable composition of Claim 1 wherein the solvent composition is a good solvent for acrylic materials, has a solubility parameter of about 8.5 to about 13.3 $(cal/cm^3)^{\frac{1}{2}}$, is weakly or moderately hydrogen bonding, swells polymerized acrylic material, and on hardening bonds and/or allows ingress of compounds that bond to polymerized acrylic material.

**0142172**

8.  The new polymerizable composition of Claim 1 wherein said acid composition is polymerizable and is present in an amount of about 1 to about 10 weight percent of the recited components, said difunctional acrylic crosslinking monomer composition is present in an amount of about 0.5 to about 20 weight percent of the recited components, said solvent compound is present in an amount of about 20 to about 60 weight percent of the recited components and is at least 25 weight percent dicyclopentenyloxyethyl methacrylate, said urethane diacrylate oligomer composition is present in an amount of about 35 to about 60 weight percent of the recited components, and said polymerization initiator system composition present in an amount of about 0.2 to about 25 weight percent of the recited components.

9. The new polymerizable composition of Claim 8 wherein said acrylic acid composition is acrylic acid or methacrylic and is present in an amount of about 3 to about 9 weight percent of the recited components, said difunctional acrylic crosslinking monomer composition is present in an amount of about 2 to about 15 weight percent of the recited components, said solvent composition compound is present in an amount of about 30 to about 50 weight percent of the recited components and is at least 30 weight percent dicyclopentenyl- oxyethyl methacrylate and at least 25 weight percent of other organic solvent composition, said urethane diacrylate oligomer composition is present in an amount of about 40 to about 50 weight percent of the recited components, and said polymerization initiator system composition comprising a visible light photoinitiator and an accelerator and present in an amount of about 0.04 to about 5 weight percent of the recited components.

10. An adhesive material comprising:
    a. polar organic composition,
    b. multifunctional vinyl crosslinking composition, and
    c. solvent composition giving said adhesive material a substantial solvent action.

11. The composition of Claim 10 comprising an initiator system activatable to cause said multifunctional vinyl crosslinking composition to crosslink.

0142172

12. The composition of Claim 11 wherein said initiator system comprising a visible light activatable compound.

13. The adhesive material of Claim 10 wherein said polar organic composition is an acid and is present in an amount of about 0.1 to about 30 weight percent of the recited components, said multifunctional vinyl crosslinking composition is difunctional acrylic crosslinking monomer composition present in an amount of about 0.1 to about 30 weight percent of the recited components, and said solvent composition is present in an amount of about 5 to about 80 weight percent of the recited components and wherein said new polymerizable composition further comprising urethane diacrylate oligomer composition present in an amount of about 20 to about 70 weight percent of the recited components and polymerization initiator system composition present in an amount of about 0.01 to about 10 weight percent of the recited components.

14. The adhesive material of Claim 10 wherein said solvent comprising dicyclopentenyloxyethyl methacrylate.

15. The adhesive material of Claim 14 wherein said solvent comprising at least about 20 weight percent dicyclopentenyloxyethyl methacrylate and at least about 25 weight percent of other organic solvent composition.

16.  The  adhesive  material  of  Claim  15  wherein said  other  organic  solvent  compound  is  chosen  from  the  group consisting  of  methyl  methacrylate  and  methylene  chloride.

17.  The  adhesive  material  of  Claim  10  wherein  the solvent  composition  is  a  good  solvent  for  acrylic  materials, has  a  solubility  parameter  of  about  8.5  to  about  13.3 $(cal/cm^3)^{\frac{1}{2}}$,  is  weakly  or  moderately  hydrogen  bonding,  swells polymerized  acrylic  material,  and  on  hardening  bonds  and/or allows  ingress  of  compounds  that  bond  to  polymerized  acrylic material.

18.  The  adhesive  material  of  Claim  17  wherein said  polar  organic  composition  is  an  acrylic  acid  composition  and  is  present  in  an  amount  of  about  1  to  about  10 weight  percent  of  the  recited  components,  said  multifunctional  vinyl  crosslinking  composition  is  a  difunctional acrylic  crosslinking  monomer  composition  and  is  present  in an  amount  of  about  0.5  to  about  20  weight  percent  of  the recited  components,  said  solvent  compound  is  present  in  an amount  of  about  20  to  about  60  weight  percent  of  the  recited components  and  is  at  least  25  weight  percent  dicyclopentenyloxyethyl  methacrylate,  and  wherein  said  adhesive material  further  comprising  urethane  diacrylate  oligomer composition  present  in  an  amount  of  about  35  to  about  60 weight  percent  of  the  recited  components,  and  polymerization initiator  system  composition  present  in  an  amount  of  about 0.1  to  about  10  weight  percent  of  the  recited  components.

- 37 -

0142172

19. The adhesive material of Claim 18 wherein said initiator system composition comprising a visible light photoinitiator.

20. The adhesive material of Claim 19 further comprising one or more selected from the group consisting of pigment fillers, adhesion promoters, thickeners, wetting agents, and stabilizers.

21. The adhesive material of Claim 15 wherein after polymerization hardening, its surface is only partially polymerized due to oxygen inhibition and is bondable with monomeric compositions.

22. A method of forming a denture comprising forming a tooth structure of ridged acrylic, coating at least a portion of said tooth structure with a bonding composition comprising:

        a. polar organic composition,

        b. multifunctional vinyl crosslinking monomer composition, and

        c. solvent composition giving said adhesive material a substantial solvent action, and

engaging an acrylic denture base composition with said coating.

23. The method of Claim 22 wherein said bonding composition comprising a radiation activatable compound.

24. The method of forming a denture of Claim 22 wherein said bonding composition comprising an initiator system comprising a visible light activatable compound.

25. The method of forming a denture of Claim 22 wherein said polar organic composition is an acid and is present in an amount of about 0.1 to about 30 weight percent of the recited components, said multifunctional vinyl crosslinking composition is difunctional acrylic crosslinking monomer composition present in an amount of about 0.1 to about 30 weight percent of the recited components, and said solvent composition is present in an amount of about 5 to about 80 weight percent of the recited components and wherein said bonding composition further comprising urethane diacrylate oligomer composition present in an amount of about 20 to about 70 weight percent of the recited components and polymerization initiator system composition present in an amount of about 0.01 to about 10 weight percent of the recited components.

26. The method of forming a denture of Claim 25 wherein said solvent comprising at least about 20 weight percent dicyclopentenyloxyethyl methacrylate and at least about 25 weight percent of other organic solvent composition.

27. A method of forming a plastic veneered dental bridge comprising forming a bridge substructure of metal, coating at least a portion of said metal with a bonding composition comprising:

    a. polar organic composition,

    b. multifunctional vinyl crosslinking monomer composition, and

    c. solvent composition giving said adhesive material a substantial solvent action, and

engaging an acrylic veneer material with said coating.

28. The method of Claim 27 wherein said bonding composition comprising a radiation activatable compound and wherein said bonding composition is cured after engagement by said acrylic veneer by exposure through said acrylic veneer by activating radiation.

29. The method of Claim 28 wherein said radiation activatable compound is a visible light activatable compound.

30. The method of Claim 27 wherein said polar organic composition is an acid and is present in an amount of about 0.1 to about 30 weight percent of the recited components, said multifunctional vinyl crosslinking composition is difunctional acrylic crosslinking monomer composition present in an amount of about 0.1 to about 30 weight percent of the recited components, and said solvent composition is present in an amount of about 5 to about 80 weight percent of the recited components and wherein said bonding composition further comprising urethane diacrylate oligomer composition present in an amount of about 20 to about 70 weight percent of the recited components and polymerization initiator system composition present in an amount of about 0.01 to about 10 weight percent of the recited components.

31. The method of Claim 30 wherein said solvent comprising at least about 20 weight percent dicyclopentenyloxyethyl methacrylate and at least about 25 weight percent of other organic solvent composition and wherein said bonding composition further comprising light opaquer agent.

32. The adhesive material of Claim 13 further comprising light opaquing agent.